# EUROPEAN PATENT SPECIFICATION

(11) **EP 3 738 557 B1**
(45) Date of publication and mention of the grant of the patent: **16.11.2022**
(21) Application number: 20172780.7
(22) Date of filing: 04.05.2020
(51) Int. Cl.: A61F 9/00, B05B 11/00, B05B 11/04

(54) **DEVICE FOR SINGLE-DOSE CONTAINERS**
VORRICHTUNG FÜR EINZELDOSISBEHÄLTER
DISPOSITIF POUR RÉCIPIENTS À DOSE UNIQUE

(30) Priority: 16.05.2019 ES 201930820 U
(43) Date of publication of application: 18.11.2020
(73) Proprietor: Garcia Conca, Víctor, 03005 Alicante (ES)
(72) Inventor: Garcia Conca, Víctor, 03005 Alicante (ES)
(74) Representative: Carlos Hernando, Borja

(56) References cited:
- ES-U- 1 217 034
- US-A- 4 550 866
- US-A1- 2009 259 204
- US-A1- 2015 088 099
- US-A1- 2016 199 226

## Description

### OBJECT OF THE INVENTION

The present invention relates to a device intended for the correct instillation of the content of single-dose containers.

The object of the invention is to provide a device formed by a single easy-to-manufacture and easy-to-use polymeric part, ensuring the correct application of the eye drop and full utilization of the product in its single-dose container.

The configuration of the developed device enables receiving the single-dose container but without contact with the content thereof, facilitating its gripping, the correct placement of the device above the eye before and during eye drop metering, and facilitating the application of the necessary pressure on the container to ensure complete metering of the content of the eye drop stored in the single-dose container.

### BACKGROUND OF THE INVENTION

In the current state of the art, are well known single-dose containers for applying different fluids, such as the eye drop instillation in the eyes.

In this sense, the use of single-dose eye drop containers is growing among the population since they ensure operating conditions, minimizing the presence of bacteria that may interfere with treatment of the eye in which the drops are applied. Another one of the main causes of the increase in use of single-dose containers relates to preventing the metering of eye drops contained in containers that have been open for a prolonged period of time.

One of the largest drawbacks of single-dose containers resides in the difficulty of using the entire product contained in its interior due to the need to apply high pressure on the walls of the container to foster its outlet.

In this sense, the use of single-dose eye drop containers is growing among the population since single-dose containers have been proven to significantly reduce contamination of the eye drops contained therein. Furthermore, as preservatives are not needed to prevent their contamination, they are less toxic than multi-dose containers on the ocular surface. Another one of the main causes of the increase in use of single-dose containers relates to the cost savings since multi-dose eye drops have an expiration date once they have been opened, whereas single-dose eye drops are opened when they are going to be used, and the rest can be left unopened.

Moreover, the main limitation to the use of these containers resides in the difficulty in being applied by users. This difficulty is due to their small gauge and the great pressure that has to be exerted to empty their content. This difficulty increases as the eye drop volume therein decreases such that over 50% of this volume is wasted.

Another one of the drawbacks provided by known single-dose containers relates to the scarce gripping surface they provide, making it difficult to hold them and, therefore, to apply the product, particularly in people with any type of condition in their hands, for example, difficulty in moving their fingers.

Finally, the need to provide a translucent material forming the single-dose container implies an increase in the difficulty of applying the content of the container in the suitable position above the eye, since it does not allow seeing the container at the eye drop application distance, which results in an incorrect application of said eye drop outside of the eye. Namely, this difficulty is accentuated in people with any type of vision anomaly or deficit.

Spanish utility model number ES1217034U, relates to a one-piece adaptor device for the ocular instillation of ophthalmic drops from a container, consisting of: an elongate adjustment body for receiving a single-dose container of ophthalmic fluid, with at least one means for holding the container; a support extension ending in an ergonomic distal end, designed to be supported on a lower area of the eyelid; and at least one projection on the adjustment body for pressing the container and helping to the squeeze out the ophthalmic fluid.

US patent application number US2009259204 describes an eye dropper with spring loaded soft tipped arms that hold open the eyelid to facilitate applying fluid eye medication drops to the eye. The eye dropper has attachment device for a bottle of liquid with a means of controllably dropping liquids into the open eye. Adjustable protrusions set maximum opening of eye and concurrently squeeze the bottle to controllably extract the eye drops.

Based on the foregoing, the applicant does not know of any devices facilitating the correct and complete metering of the content stored in a single-dose container.

### DESCRIPTION OF THE INVENTION

The metering device for single-dose containers, for example, for metering eye drops, has a configuration which enables the reception of a single-dose container such that it prevents the part of the container which allows the fluid or eye drop to come out from directly contacting the metering device itself.

More specifically, the metering device for single-dose containers consists of side arms emerging from a perforated base, with the main function of the side arms being to facilitate the correct placement of the single-dose container inside the dispenser and, in turn, help with the instillation of the fluid contained in the metering device itself.

Said arms have pressure means arranged on their inner part, the function of which is to compress the single-dose eye drop container by the user applying external pressure by means of a lever effect, thereby facilitating complete extraction of the content from single-dose containers. The pressure means are formed by wedges arranged in the inner part of the side arms or, optionally, by a plurality of rounded protrusions with an effect equivalent to the effect resulting from the presence of the mentioned wedges.

Advantageously, the metering device for single-dose containers has gripping means, namely by way of a rough surface, located on the outer part of its side arms, facilitating the gripping thereof by the user.

Additionally, the metering device object of the invention has a perforated base, having a side opening to facilitate placement of the single-dose container in the metering device itself. The side opening of the base facilitates the correct placement the single-dose container, such that its arrangement on the metering device allows the drops of fluid to be instilled to be led through the perforation present in the base of the metering device with great ease and control by the user pressing the container.

Additionally, the perforation present in the base of the metering device is characterized by having an inclined surface along the edge of its perimeter which advantageously even further facilitates coupling the nozzle defined by breaking the seal of the single-dose container on the metering device.

Advantageously, the metering device is formed by a colored polymer material for the purpose of facilitating for the user the correct positioning of the metering device above the eye. During the operation of handling and instilling the eye drop, the color of the material of the metering device thereby favors it being easy to see by the user and being correctly placed above the central part of the eye.

### DESCRIPTION OF THE DRAWINGS

To complement the description provided below and for the purpose of helping to better understand the features of the invention according to a preferred practical embodiment thereof, a set of drawings is attached as an integral part of said description where the following is depicted in an illustrative and non-limiting manner:
Figure 1 shows a perspective view of a first embodiment of the metering device object of the invention.
Figure 2 shows a perspective view of the metering device object of the invention wherein the gripping means have a shape other than that shown in Figure 1.
Figure 3 shows a front view of the metering device, wherein its side opening can be seen.
Figure 4 shows a top view of the metering device.
Figure 5 shows a view of the metering device depicted together with the single-dose container arranged therein.
Figure 6 shows a perspective view of a second embodiment of the metering device object of the invention.
Figure 7 shows a top view of the second embodiment of the metering device object of the invention.

### PREFERRED EMBODIMENT OF THE INVENTION

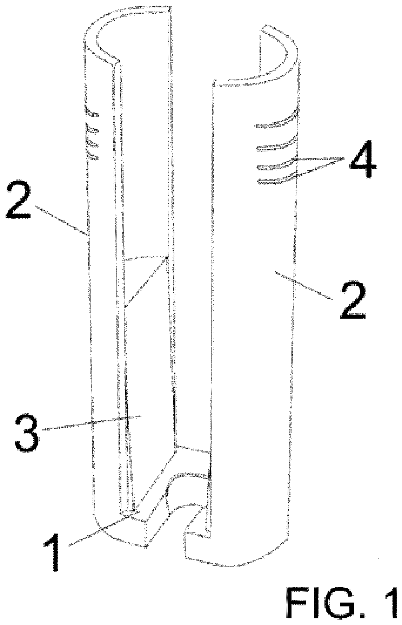

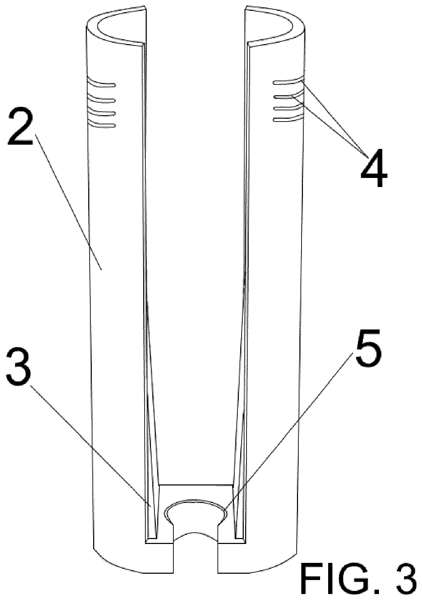

In view of Figures 1, 2, and 3, there can be observed the metering device formed by a perforated base (1) from which there preferably emerge two side arms (2), favoring the correct application of the eye drop contained in a single-dose container.

The user thereby applies controlled pressure on the side arms (2) which is enhanced by the lever effect for control and complete metering of the eye drop contained in the single-dose container.

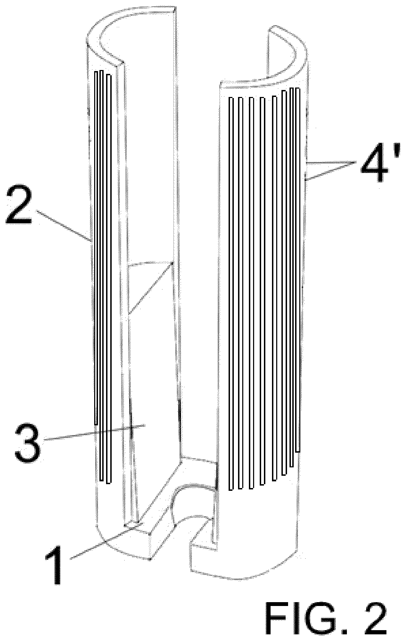

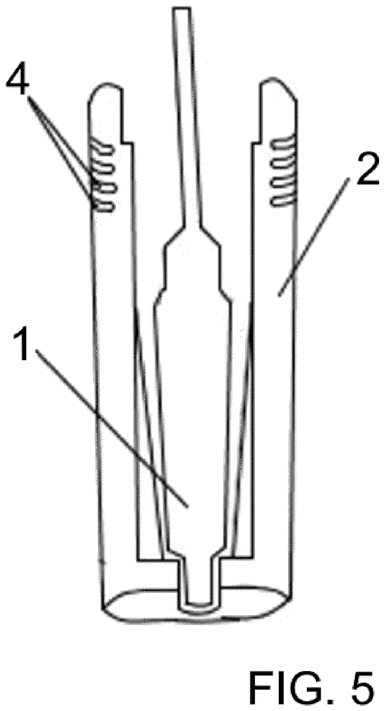

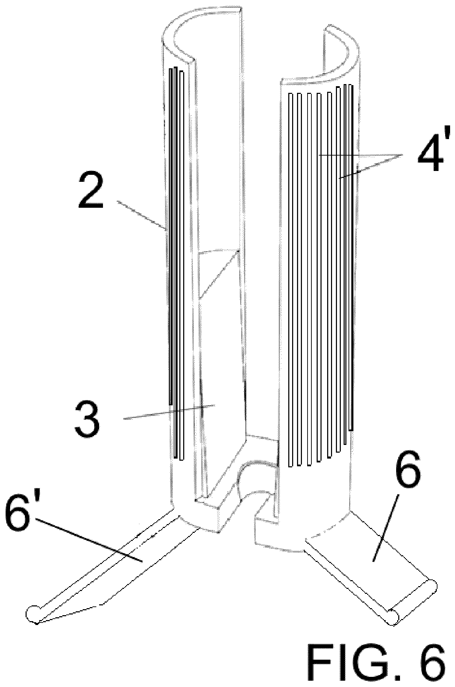

The perforated base (1) has an opening (1') to facilitate the single-dose container entering from the side and being optimally placed on the applicator. Additionally, the side arms (2) have gripping means located on their outer part to foster suitable gripping by the user. As can be observed in Figures 1 and 2, the gripping means are formed by a plurality of either horizontal projections (4), as depicted in Figures 1, 3, and 5, or formed by vertical projections (4') extending across the outer surface of the side arms (2), as depicted in Figures 2 and 6.

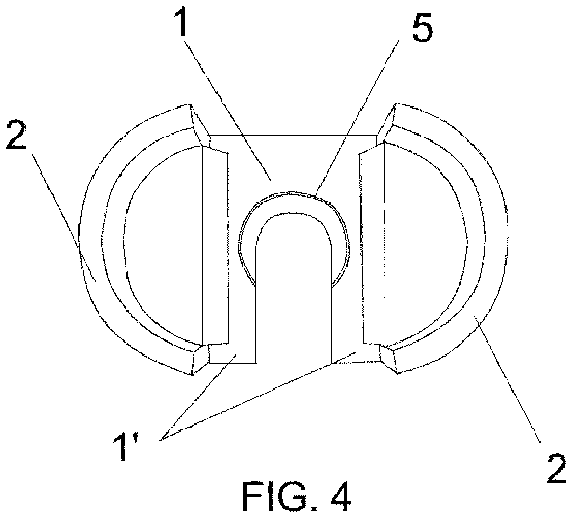

Additionally, it can be observed in Figures 2, 3, and 4 that the perforated base (1) has an inclined surface (5) along the edge of its perimeter simulating the termination of the single-dose container, thereby facilitating complete coupling

Complementarily in Figures 2, 3, and 4, it can be observed that the metering device has pressure means (3) formed by wedges arranged in the inner part of each side arm (2) to facilitate the lever effect.

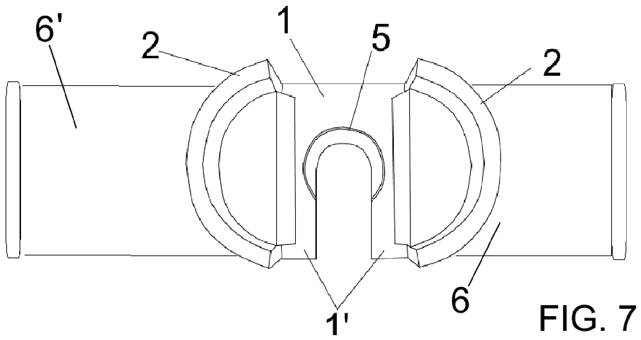

In a second preferred embodiment depicted in Figures 6 and 7, the metering device incorporates support means (6) and (6'), preferably having a planar rectangular body emerging from of the perforated base (1) in the opposite direction with respect to the side arms (2) and facilitating the effectiveness of the centered placement of the metering device above the ocular area.

Advantageously, the support means (6) and (6') are slightly inclined and have different lengths to facilitate support on the orbital rim. Namely, the end of the support means having a smaller length (6) is located on the upper orbital rim, whereas the end of the support means having a greater length (6') is supported on the lower orbital rim. The instillation of the drops of fluid contained in the single-dose container arranged in the metering device is thereby facilitated with a high effectiveness and control by the user pressing the container.

In a preferred embodiment of the invention depicted in the figures attached to this specification, it can be observed that the side arms (2) of the present invention have a rounded shape. Nevertheless, in another embodiment not depicted in the figures attached to this description, the side arms (2) have a planar or rectangular shape for the purpose of providing a handling surface for the user offering greater control for metering the eye drops contained in single-dose containers.

## Claims

1. Device for the complete instillation of drops of fluid contained in a single-dose container,
**characterized in that** it comprises:
- a base with a perforation (1), and
- two side arms (2) emerging substantially perpendicular from the base, and comprising pressure means (3) consisting of a wedge (3) arranged along the inner part of each side arm (2), each wedge (3) having one thick end tapering to a thinner end along the arm, said thick end placed on the perforated base (1), and each wedge having an inclined surface facing the opposite wedge, said means (3) being adapted to compress the container between said means (3) by a user applying external pressure on the arms.

2. The device, according to claim 1, **characterized in that** comprises two inclined support means (6, 6') with different lengths emerging from the base with the perforation (1) in an opposite direction with respect to the side arms (2).

3. The device according to claim 1, **characterized in that** the inside surface (5) of the perforation in the base (1) is inclined along its perimeter.

4. The device, according to the preceding claims, **characterized in that** the side arms (2) have gripping means (4) located on their outer part.

5. The device, according to claim 1, **characterized in that** the base with the perforation (1) has a side opening (1'), between the side arms (2) and the support means (6, 6') to facilitate the placement of a single-dose container.

6. The device, according to claim 1, **characterized in that** the outer part of the side arms has a rounded shape.

7. The device, according to claim 1, **characterized in that** it is formed by a colored polymer material.

8. System for the instillation of the content of single-dose containers, comprising a device according to claims 1 to 7 and a single-dose container placed between the two arms and inside the perforation of the base.

## Patentansprüche

1. Vorrichtung für eine vollständige Einträufelung von Fluidtropfen, die in einem Einzeldosisbehälter enthalten sind,
**dadurch gekennzeichnet, dass** sie Folgendes umfasst:
- eine Basis mit einer Perforation (1) und
- zwei Seitenarme (2), die im Wesentlichen senkrecht aus der Basis herausragen und Druckmittel (3) umfassen, die aus einem Keil (3) bestehen, der entlang des inneren Teils jedes Seitenarms (2) angeordnet ist, wobei jeder Keil (3) ein dickes Ende aufweist, das sich entlang des Arms zu einem dünneren Ende verjüngt, wobei das dicke Ende auf der perforierten Basis (1) platziert ist und jeder Keil eine geneigte Fläche aufweist, die dem gegenüberliegenden Keil zugewandt ist, wobei die Mittel (3) angepasst sind, den Behälter durch die Mittel (3) zusammenzudrücken, indem ein Benutzer einen äußeren Druck auf die Arme ausübt.

2. Vorrichtung nach Anspruch 1, **dadurch gekennzeichnet, dass** zwei geneigte Stützmittel (6, 6') mit unterschiedlichen Längen umfasst, die aus der Basis herausragen, wobei die Perforation (1) mit Bezug auf die Seitenarme (2) eine entgegengesetzte Richtung aufweist.

3. Vorrichtung nach Anspruch 1, **dadurch gekennzeichnet, dass** die Innenfläche (5) der Perforation in der Basis (1) entlang ihres Umfangs geneigt ist.

4. Vorrichtung nach einem der vorhergehenden Ansprüche, **dadurch gekennzeichnet, dass** die Seitenarme (2) Greifmittel (4) aufweisen, die sich an deren Außenteil befinden.

5. Vorrichtung nach Anspruch 1, **dadurch gekennzeichnet, dass** die Basis mit der Perforation (1) eine Seitenöffnung (1') zwischen den Seitenarmen (2) und den Stützmitteln (6, 6') aufweist, um die Platzierung eines Einzeldosisbehälters zu ermöglichen.

6. Vorrichtung nach Anspruch 1, **dadurch gekennzeichnet, dass** der Außenteil der Seitenarme eine gerundete Form aufweist.

7. Vorrichtung nach Anspruch 1, **dadurch gekennzeichnet, dass** sie aus einem farbigen Polymermaterial gebildet ist.

8. System für die Einträufelung des Inhalts von Einzeldosisbehältern, das eine Vorrichtung nach Anspruch 1 bis 7 und einen Einzeldosisbehälter, der zwischen den beiden Armen und in der Perforation der Basis platziert ist, umfasst.

## Revendications

1. Dispositif pour l'instillation complète de gouttes de fluide contenues dans un récipient unidose,
**caractérisé en ce qu'**il comprend :
- une base avec une perforation (1), et
- deux bras latéraux (2) émergeant sensiblement perpendiculairement de la base, et comprenant des moyens de pression (3) consistant en une cale (3) agencée le long de la partie interne de chaque bras latéral (2), chaque cale (3) ayant une extrémité épaisse se rétrécissant en une extrémité plus mince le long du bras, ladite extrémité épaisse placée sur la base perforée (1), et chaque cale ayant une surface inclinée faisant face à la cale opposée, lesdits moyens (3) étant adaptés pour compresser le récipient entre lesdits moyens (3) par un utilisateur appliquant une pression externe sur les bras.

2. Dispositif, selon la revendication 1, **caractérisé en ce que** comprend deux moyens de support inclinés (6, 6') avec des longueurs différentes émergeant de la base avec la perforation (1) dans une direction opposée par rapport aux bras latéraux (2).

3. Dispositif selon la revendication 1, **caractérisé en ce que** la surface intérieure (5) de la perforation dans la base (1) est inclinée le long de son périmètre.

4. Dispositif, selon les revendications précédentes, **caractérisé en ce que** les bras latéraux (2) ont des moyens de préhension (4) situés sur leur partie extérieure.

5. Dispositif, selon la revendication 1, **caractérisé en ce que** la base avec la perforation (1) a une ouverture latérale (T), entre les bras latéraux (2) et les moyens de support (6, 6') pour faciliter le placement d'un récipient unidose.

6. Dispositif, selon la revendication 1, **caractérisé en ce que** la partie extérieure des bras latéraux a une forme arrondie.

7. Dispositif, selon la revendication 1, **caractérisé en ce qu'**il est formé par un matériau polymère coloré.

8. Système pour l'instillation du contenu de récipients unidoses, comprenant un dispositif selon les revendications 1 à 7 et un récipient unidose placé entre les deux bras et à l'intérieur de la perforation de la base.
